# EUROPEAN PATENT SPECIFICATION

(11) **EP 3 762 017 B1**
(45) Date of publication and mention of the grant of the patent: **17.09.2025**
(21) Application number: 19764435.4
(22) Date of filing: 08.03.2019
(51) Int. Cl.: A61K 38/26, A61P 3/04, A61K 8/64, A61Q 19/06

(54) **GLP-1 ANALOGUE BEINAGLUTIDE COMPOSITION FOR TREATING OBESITY AND WEIGHT MANAGEMENT**
GLP-1-ANALOG BEINAGLUTIDE ZUSAMMENSETZUNG ZUR BEHANDLUNG VON ADIPOSITAS UND ZUM GEWICHTSMANAGEMENT
COMPOSITION D'ANALOGUE DE GLP-1 BEINAGLUTIDE POUR LE TRAITEMENT DE L'OBÉSITÉ ET LA GESTION DU POIDS

(30) Priority: 09.03.2018 CN 201810198521
(43) Date of publication of application: 13.01.2021
(73) Proprietor: Shanghai Benemae Pharmaceutical Corporation, Shanghai 200321 (CN)
(72) Inventor: YU, Zhiwen, Shanghai 200321 (CN); ZUO, Yajun, Shanghai 200321 (CN); XIA, Jing, Shanghai 200321 (CN); WANG, Shichuang, Shanghai 200321 (CN); QIAN, Lifen, Shanghai 200321 (CN)
(74) Representative: Gulde & Partner
(86) International application number: PCT/CN2019/077541
(87) International publication number: WO 2019/170153

(56) References cited:
- EP-A2- 1 529 534
- WO-A1-98/19698
- CN-A- 1 950 078
- CN-A- 104 277 103
- CN-A- 107 462 559
- NAUCK M ET AL: "Long-term efficacy and safety comparison of liraglutide, glimepiride and placebo, all in combination with metformin in type 2 diabetes: 2-year results from the LEAD-2 study", DIABETES, OBESITY AND METABOLISM, 11 October 2012 (2012-10-11), pages 204 - 212, XP055839972, DOI: 10.1111/dom.12012
- M. NAUCK ET AL: "Efficacy and Safety Comparison of Liraglutide, Glimepiride, and Placebo, All in Combination With Metformin, in Type 2 Diabetes: The LEAD (Liraglutide Effect and Action in Diabetes)-2 study", DIABETES CARE, vol. 32, no. 1, 1 January 2009 (2009-01-01), pages 84 - 90, XP055041024, ISSN: 0149-5992, DOI: 10.2337/dc08-1355
- BROWN EMILY ET AL: "Newer GLP-1 receptor agonists and obesity-diabetes", PEPTIDES, vol. 100, 1 February 2018 (2018-02-01), AMSTERDAM, NL, pages 61 - 67, XP055840009, ISSN: 0196-9781, DOI: 10.1016/j.peptides.2017.12.009
- DANDONA PARESH ET AL: "Incretins: Beyond type 2 diabetes", vol. 20, 24 January 2018 (2018-01-24), GB, pages 59 - 67, XP055840002, ISSN: 1462-8902, Retrieved from the Internet <URL:https://api.wiley.com/onlinelibrary/tdm/v1/articles/10.1111%2Fdom.13153> DOI: 10.1111/dom.13153
- JIA YING CHEANG ET AL: "Glucagon-Like Peptide-1 (GLP-1)-Based Therapeutics: Current Status and Future Opportunities beyond Type?2 Diabetes", CHEMMEDCHEM COMMUNICATIONS, vol. 13, no. 7, 28 February 2018 (2018-02-28), DE, pages 662 - 671, XP055730475, ISSN: 1860-7179, DOI: 10.1002/cmdc.201700781
- ZHANG Y. L. ET AL: "Beinaglutide showed significant weight-loss benefit and effective glycaemic control for the treatment of type 2 diabetes in a real-world setting: a 3-month, multicentre, observational, retrospective, open-label study", vol. 5, no. 4, 17 June 2019 (2019-06-17), pages 366 - 375, XP055828481, ISSN: 2055-2238, Retrieved from the Internet <URL:https://onlinelibrary.wiley.com/doi/full-xml/10.1002/osp4.342> DOI: 10.1002/osp4.342
- JURIS J. MEIER ET AL.: "Glucagon-like peptide 1 as a regulator of food intake and body weight: therapeutic perspectives", EUROPEAN JOURNAL OF PHARMACOLOGY, vol. 440, 31 December 2002 (2002-12-31), pages 269 - 279, XP001113094, doi:10.1016/S0014-2999(02)01434-6
- L VAN BLOEMENDAAL ET AL.: "Effects of glucagon-like peptide 1 on appetite and body weight: focus on the CNS", JOURNAL OF ENDOCRINOLOGY, vol. 221, no. 1, 9 December 2013 (2013-12-09), pages T1 - T16, XP055637385

## Description

### Background

GLP-1 is an insulinotropic peptide acting on GLP-1 receptor distributed through the whole body, and particularly on pancreatic insulin-secreting β cells. This type of hormones can promote insulin secretion, and also stimulates insulin signaling independently, resulting a glucose hypoglycemic effect. Instability of GLP-1 includes two general aspects, i.e., physical and chemical aspects. Physical instability includes, for example, denaturation, surface adsorption, aggregation, precipitation, gelatination, and the like, and chemical instability includes, for example, hydrolysis, deamination, oxidization, racemization, isomerization, β-elimination, disulfide bond exchange, and the like. Instability not only shortens the shelf life but also affects the efficacy of GLP-1 compositions. To solve the instability problem, either nanoparticles are used to deliver GLP-1 or GLP-1 is modified to improve stability. However, nanoparticle delivery increases the production cost and formulation complexity, and modification of GLP-1 is often associated with increased toxicity. See Lee, Basic Clin. Pharmacol. Toxicol. 118(3): 173-180 (2016).

In WO 98/19698 A1, it is disclosed the use of GLP-1 in treatment of type-2 diabetes overweight via subcutaneous continuous infusion. In EP 1 529 534 A2, it is disclosed the use of different GLP-1 analogues (including GLP-1) for treatment of obesity, wherein the route of administration, the dose and the dosing regime have to be chosen from a large number of options.

Accordingly, a composition containing GLP-1 in its natural, unmodified form with increased stability and improved efficacy at a low dose is highly desirable. The technology and methods disclosed herein satisfy this need in the art.

### Summary

The present invention is directed to subject matter as defined in the appended claims. For the purpose of the disclosure of the present specification, any references to methods of treatment by therapy or surgery or in vivo diagnosis methods provided herein are to be interpreted as references to compounds, pharmaceutical compositions and medicaments for use in those methods.

In one aspect, the present disclosure provides a method of treating obesity by administering to a subject suffering from obesity or overweight a therapeutically effective amount of a composition comprising a GLP-1 peptide ( GLP-1 (7-36)), the therapeutically effective amount of the GLP-1 peptide is from 0.11 mg/day to 0.66 mg/day (e.g., in a human subject). In some embodiments, the GLP-1 peptide is a recombinant human GLP-1 peptide. In some embodiments, the GLP-1 peptide is a chemically synthesized GLP-1 peptide. In some embodiments, the composition comprising a GLP-1 peptide (the GLP-1 composition) is administered by parenteral injection. In some embodiments, the GLP-1 composition is administered by intravenous injection, intramuscular injection, or subcutaneous injection. In some embodiments, the GLP-1 composition is administered for an extended period of time, up to 1 year, up to 2 years, up to 3 years, up to 4 years, up to 5 years, up to 6 years, up to 7 years, up to 8 years, up to 9 years, up to 10 years, up to 11 years, up to 12 years, up to 13 years, up to 14 years, up to 15 years, up to 16 years, up to 17 years, up to 18 years, up to 19 years, up to 20 years, up to 21 years, up to 22 years, up to 23 years, up to 24 years, up to 25 years, up to 26 years, up to 27 years, up to 28 years, up to 29 years, up to 30 years, or up to 31 years by continuous or periodic administration.

In some embodiments, the therapeutically effective amount of the GLP-1 composition for treating obesity in a female subject is lower than that in a male subject. In some embodiments, the therapeutically effective amount of the GLP-1 for treating obesity in a female subject is at least 10%, at least 20%, at least 30%, at least 40%, at least 50%, at least 60%, or at least 70% lower than that for treating obesity in a male subject to achieve the same or similar therapeutic effects. In some embodiments, the therapeutically effective amount of the GLP-1 for treating obesity in a female subject is from 0.11 mg/day to 0.66 mg/day (e.g., in human subjects). In some embodiments, the therapeutically effective amount of the GLP-1 for treating obesity in a male subject is from 0.33 mg/day to 0.66 mg/day (e.g., in human subjects).

In another aspect, the disclosure relates to a cosmetic method of managing body weight by administering to a subject in need thereof a therapeutically effective amount of a composition comprising GLP-1 (7-36), preferably a recombinant human GLP-1 (7-36), the therapeutically effective amount of the GLP-1 composition is from 0.11 mg/day to 0.66 mg/day (e.g., in a human subject). In some embodiments, the GLP-1 peptide is a chemically synthesized GLP-1 peptide. In some embodiments, the GLP-1 composition is administered by parenteral injection. In some embodiments, the GLP-1 composition is administered by intravenous injection, intramuscular injection, or subcutaneous injection. In some embodiments, the GLP-1 composition is administered for an extended period of time, up to 1 year, up to 2 years, up to 3 years, up to 4 years, up to 5 years, up to 6 years, up to 7 years, up to 8 years, up to 9 years, up to 10 years, up to 11 years, up to 12 years, up to 13 years, up to 14 years, up to 15 years, up to 16 years, up to 17 years, up to 18 years, up to 19 years, up to 20 years, up to 21 years, up to 22 years, up to 23 years, up to 24 years, up to 25 years, up to 26 years, up to 27 years, up to 28 years, up to 29 years, up to 30 years, or up to 31 years by continuous or periodic administration. In some embodiments, the body weight loss of the subject the GLP-1 composition is less than 100%, less than 95%, less than 90%, less than 85%, less than 80%, or less than 75% of the body weight loss of the subject receiving the GLP-1 composition. In some embodiments, the therapeutically effective amount of the GLP-1 for weight management in a female subject is from 0.11 mg/day to 0.66 mg/day. In some embodiments, the therapeutically effective amount of the GLP-1 for weight management in a male subject is from 0.33 mg/day to 0.66 mg/day.

### Brief Description of the Drawings

Figure 1 shows the rate of gastric remains of carbon powder in male and female rats received various dosages of Beinaglutide, comparing to buffer only control and liraglutide.
Figure 2 shows the propelling rate of carbon powder in the small intestine of male and female rats received various dosages of Beinaglutide, comparing to buffer only control and liraglutide.

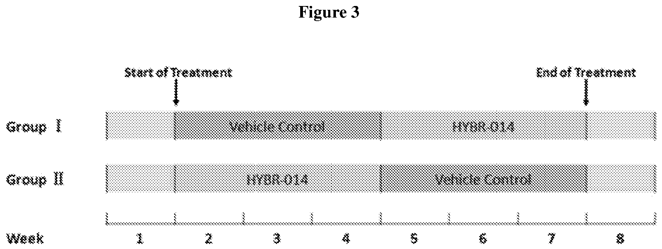

Figure 3 shows an experimental design of treating obesity in cynomolgus monkeys. Four diet induced obesity cynomolgus monkeys were divided into two treatment groups, each group having one male monkey and one female monkey. During week 1 of the study period, the food intake, body weight, BMI and body composition of each animal were measured and recorded as baseline indexes. During the treatment period (week 2 through week 7), Group 1 animals received subcutaneous administration of blank control in the first three weeks (week 2 through week 4) and Beinaglutide in the second three weeks (week 5 through week 7), respectively. Group 2 animals received subcutaneous administration of Beinaglutide in the first three weeks (week 2 through week 4) and blank control in the second three weeks (week 5 through week 7), respectively. Administration of Beinaglutide or blank control was stopped in week 8 for both groups. The efficacy and reversibility of Beinaglutide treatment were evaluated based on food intake, body weight, BMI, and body composition at week 4, week 7 and week 8, compared to baseline levels.

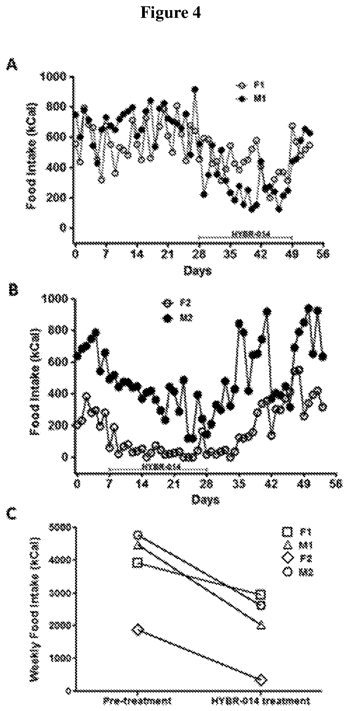

Figures 4A-4C demonstrate that Beinaglutide treatment reduced food intake in obesity monkeys. Figure 4A demonstrates the daily calories intake of each cynomolgus monkey in Group 1. Figure 4B demonstrates the daily calories intake of each cynomolgus monkey in Group 2. Figure 4C shows the average weekly food intake for each animal before and after Beinaglutide treatment. Before treatment: Group 1, week 1 through week 4, and Group 2, week 1; during treatment: Group 1, week 5 through week 7, and Group 2, week 2 through week 4. During treatment, the average weekly food intake for each animal exhibited significant decrease.
Figures 5A-5B demonstrate the decrease of body weight of obesity monkeys. Figure 5A demonstrates the percentage of body weight change of each animal during the 8-week study period. Treatment with Beinaglutide is shown as solid lines and blank control is shown in dotted lines. Figure 5B demonstrates the body weight of each animal before and after Beinaglutide treatment. All animal groups exhibited significant body weight loss after Beinaglutide treatment (n = 4).
Figures 6A-6C show the effect of Beinaglutide in combination with glipizide or metformin on subjects. Figure 6A shows the change in body mass index at week 12 of the 5A test group. Figure 6B shows changes in body mass index during the 2nd to 12th week of the 5B trial (four groups of data for each dose, showing body mass index for 2 weeks, 4 weeks, 8 weeks, and 12 weeks from left to right). Figure 6C is a graph showing the chronological changes in body weight during the trial of patients in overweight and obese patients in the trial 5C.
Figure 7 shows the rate of gastric remains of carbon powder in male and female rats received various dosages of Beinaglutide from 10 µg/kg to 60 µg/kg, comparing to buffer only control.
Figure 8 shows the propelling rate of carbon powder in the small intestine of male and female rats received various dosages of Beinaglutide from 10 µg/kg to 60 µg/kg, comparing to buffer only control.
Figure 9 shows the change of body weight of the mice on high-fat diet and low-fat diet over a period of 15 weeks.
Figure 10 shows the food intake of the mice on high-fat diet and low-fat diet over a period of 15 weeks.
Figure 11 shows the results of intraperitoneal glucose tolerance test (IPGTT) at the end of special diet to confirm successful induction of insulin resistance and obesity.
Figure 12A shows the rate of gastric remains of carbon powder in male DIO mice received various dosages of Beinaglutide from 15 µg/kg to 100 µg/kg, comparing to buffer only control. Figure 12B shows the propelling rate of carbon powder in the small intestine of male DIO mice received various dosages of Beinaglutide from 15 µg/kg to 100 µg/kg, comparing to buffer only control. *P<0.05, **P<0.1.

### Detailed Description

Surprisingly, at a significantly lower dose, the GLP-1 composition described herein achieves pharmacokinetics significantly different from the liraglutide composition in treating overweight and obesity. Additionally, the therapeutically effective amount of the GLP-1 composition for a female subject is significantly lower than the therapeutically effective amount of the GLP-1 composition for a male subject to achieve the same or similar therapeutic effects.

The World Health Organization (WHO) has listed obesity as a disease since 1948. The worldwide occurrence of obesity has more than doubled since 1980. Ng et al., Lancet. 384: 766-781 (2014). Overweight or obesity is described as an abnormal or overly accumulation of body fat due to long-term energy intake exceeding energy expenditure. Overweight and obesity are significant risk factors for non-contagious diseases such as cardiovascular diseases, type 2 diabetes (T2DM), skeletal muscle abnormalities, and certain cancers. Van Bloemendaal et al., J. Endocrinol. 221: T1-T16 (2014). These diseases and conditions associated with overweight or obesity place a significant financial burden on public healthcare.

The native form of GLP-1is secreted by intestinal L-cells after a meal, and is a strong peptide stimulator of insulin secretion. GLP-1 is a potential therapy for type 2 diabetes. Holst, Physiol. Rev. 87: 1409-1439 (2007). GLP-1 has two active forms, GLP-1 (7-36)-NH₂ and GLP-1 (7-37). Upon entry into circulation, GLP-1 is rapidly degraded by dipeptidyl peptidase-4 (DPP4), resulting in a short half-life of about 2 minutes. Kieffer et al., Endocrinology 136: 3585-3596 (1995). Besides its effect on controlling blood sugar, GLP-1 and its analogs were found to have effects on inducing body weight loss, slowing stomach emptying, and increasing satiety. Monami et al., Exp. Diabetes Res.2012: 672658 (2012). FDA approved Saxenda (liraglutide 3 mg) in December 2014, which is the first NDA application for GLP-1 analogs, and which is a long-term weight management supplemented with low-calorie diet and increase of physical activity for obesity adults having at least one disease or condition associated with overweight, such as type 2 obesity.

In the context of this disclosure, the phrase a "therapeutically effective amount" or an "effective amount" of a pharmaceutical composition comprising GLP-1 as used herein is an amount of the pharmaceutical composition that produces a desired therapeutic effect in a subject, such as treating obesity or overweight, weight management, inducing weight loss, etc. In certain embodiments, the therapeutically effective amount is an amount of the pharmaceutical composition that yields maximum therapeutic effect. In other embodiments, the therapeutically effective amount yields a therapeutic effect that is less than the maximum therapeutic effect. For example, a therapeutically effective amount may be an amount that produces a therapeutic effect while avoiding one or more side effects associated with a dosage that yields maximum therapeutic effect. In some embodiments, a therapeutically effective amount is the minimal amount that produces a therapeutic effect. A therapeutically effective amount for a particular composition will vary based on a variety of factors, including but not limited to the characteristics of the therapeutic composition (e.g., activity, pharmacokinetics, pharmacodynamics, and bioavailability), the physiological condition of the subject (e.g., age, body weight, sex, disease type and stage, medical history, general physical condition, responsiveness to a given dosage, and other present medications), the nature of any pharmaceutically acceptable carriers, excipients, and preservatives in the composition, and the route of administration. One skilled in the clinical and pharmacological art will be able to determine a therapeutically effective amount through routine experimentation, namely by monitoring a subject's response to administration of the pharmaceutical composition and adjusting the dosage accordingly. For additional guidance, see, e.g., Remington: The Science and Practice of Pharmacy, 22nd Edition, Pharmaceutical Press, London, 2012, and Goodman & Gilman's The Pharmacological Basis of Therapeutics, 12th Edition, McGraw-Hill, New York, NY, 2011, the entire disclosures of which are incorporated by reference herein.

In some embodiments, a therapeutically effective amount of a pharmaceutical composition comprising GLP-1 disclosed herein is in the range from 0.11 mg/day to 0.66 mg/day of GLP-1 (e.g., in a human subject). In some embodiments, a therapeutically effective amount of a pharmaceutical composition comprising GLP-1 disclosed herein for a male subject is from 0.33 mg/day to 0.66 mg/day of GLP-1 (e.g., in a human subject). In some embodiments, a therapeutically effective amount of a pharmaceutical composition comprising GLP-1 disclosed herein for a female subject is from about 0.11 mg/day to 0.66 mg/day of GLP-1 (e.g., in a human subject).

The terms "treat," "treating," and "treatment" as used herein with regard to a condition refers to alleviating the condition partially or entirely, preventing the condition, decreasing the likelihood of occurrence or recurrence of the condition, slowing the progression or development of the condition, or eliminating, reducing, or slowing the development of one or more symptoms associated with the condition.

As used herein, the term "subject" refers to a mammalian subject, preferably human. A "subject in need thereof" refers to a subject who has been diagnosed with obesity or overweight, who is at a risk of weight gain, or who desires to control weight gain or induce weight loss. The phrases "subject" and "patient" can be used interchangeably herein.

As used herein, the term "comprising" with regard to a composition or method means that the composition or method includes at least the recited elements. The term "consisting essentially of" means that the composition or method includes the recited elements, and may further include one or more additional elements that do not materially affect the novel and basic characteristics of the composition or method. For example, a composition consisting essentially of recited elements may include those recited elements plus one or more trace contaminants from the isolation and purification method, pharmaceutically acceptable carriers such as phosphate buffered saline, preservatives, and the like. The term "consisting of" means the composition or method includes only the recited elements. Embodiments defined by each of the transitional terms are within the scope of this invention.

### Pharmaceutical composition comprising GLP-1

The GLP-1 composition disclosed herein comprises a recombinant human GLP-1 (7-36) peptide having the sequence of: His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg (SEQ ID NO: 1), e.g., referred to as Beinaglutide in this disclosure. Beinaglutide has a molecular formula of C₁₄₉H₂₂₅N₃₉O₄₆, and a molecular weight of 3298.7. This peptide is essentially the same as the active form of circulating GLP-1 except for the endogenous amidation, where NH₂ in the natural form is replaced by OH group in the recombinant peptide. Beinaglutide includes a C-terminal free carboxyl group, similar to glycine-extended GLP-1 (7-37). Not in accordance with the claimed invention, GLP-1 (7-35) or GLP-1 (7-37) can be used in the disclosed technology. The sequences of GLP-1 (7-35) and GLP-1 (7-37) are as follows: His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly (SEQ ID NO: 2), and His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Gly (SEQ ID NO: 3).

In clinical pharmacokinetic studies using monkey models, Beinaglutide demonstrated a half-life of about 15-30 minutes when adminitered subcutaneously. In clinical trial phase III in China for treating diabetes, 346 diabetes patients who received Beinaglutide (0.2 mg, TID) for 12 weeks demonstrated significant body weight loss as well as a decrease of glycated hemoglobin level in the blood, compared to a control group of 113 patients. The most common side effects for these patients were nausea and vomitting, however, these side effects were temporary and not worse than other similar GLP-1 analog therapies.

Beinaglutide can be isolated after expression in E. coli, and subsequently formulated into an injectable formulation. In the pharmaceutical composition of the present disclosure, the GLP-1 peptide has a concentration of about 0.1 to about 20 mg/mL, about 0.2 to about 10 mg/mL, about 0.05 to about 0.5 mg/mL, about 0.5 to about 5 mg/mL, about 1 to about 5 mg/mL, or about 2 to about 4 mg/mL. Unless specifically defined otherwise, "about" refers to the difference from a stated numerical value in a range of ±10%. For example, in the formulation of the present disclosure, the GLP-1 concentration is up to 2 mg/ml, and can be stored in dark for about 2 years at 2-8 °C.

The pharmaceutical composition of the present disclosure has a pH value of about 3.5 to about 5.0, about 3.5 to about 4.5, about 3.6 to about 4.2, about 3.6 to about 4.1, about 3.6 to about 4.0, or about 3.6 to about 3.9. At this pH range, stability of the GLP-1 composition is improved, and the composition can be stored in dark for about 2 years or more at 2 to 8 °C.

The GLP-1 composition disclosed herein may contain one or more buffer salt to maintain the desired pH range of the composition, including sodium acetate, acetic acid, etc. Additional ingredients for the GLP-1 composition may include one or more of a preservative (e.g., phenols and the like), an isotonic agent (e.g., mannitol, propylene glycol, and the like), and a dissolution enhancer (e.g., propylene glycol and the like).

In some embodiments, Beinaglutide can be administered to patients suffering from overweight or obesity by subcutaneous injection to reduce body weight. Other suitable injection routes can be used as well. The effects of Beinaglutide on controlling or reducing body weight are demonstrated by the working examples.

### Prophylactic and/or therapeutic use of the GLP-1 composition

The present disclosure provides use of the pharmaceutical composition comprising GLP-1 for treating or preventing various conditions, including obesity, bulimia, and/or overweight. This disclosure also relates to the use of the pharmaceutical composition comprising GLP-1 for weight management, slowing gastric emptying, and/or improving satiety.

According to the standard published by Centers for Disease Control and Prevention (CDC) and World Health Organization (WHO), a BMI below 18.5 indicates underweight, a BMI between 18.5 and 24.9 indicates normal or healthy weight, a BMI between 25.0 and 29.9 indicates overweight, and a BMI at 30.0 or above indicates obesity.

### Treatment of obesity

In one aspect, provided herein is a method of treating obesity comprising administering to the subject a therapeutically effective amount of the pharmaceutical composition comprising GLP-1, the therapeutically effective amount is in the range from 0.11 mg/day to 0.66 mg/day of GLP-1 (e.g., in a human subject).

### Treatment of bulimia

In one aspect, provided herein is a method of treating bulimia comprising administering to the subject a therapeutically effective amount of the pharmaceutical composition comprising GLP-1, the therapeutically effective amount is in the range from 0.11 mg/day to 0.66 mg/day of GLP-1 (e.g., in a human subject).

### Body weight management

In another aspect, provided herein is a method of managing body weight in a subject comprising administering to the subject a therapeutically effective amount of the pharmaceutical composition comprising GLP-1 such that the body mass index (BMI) is decreased by at least 0.5%, at least 1%, at least 2%, or at least 3%, the therapeutically effective amount is in the range from 0.11 mg/day to 0.66 mg/day of GLP-1 (e.g., in a human subject). Alternatively, provided herein is a method of managing body weight in a subject comprising administering to the subject a therapeutically effective amount of the pharmaceutical composition comprising GLP-1 such that the body weight is decreased by at least 2%, at least 5%, at least 10%, at least 15%, at least 20%, at least 25%, at least 30%, at least 35%, at least 40%, at least 45%, or at least 50%, the therapeutically effective amount is in the range from 0.11 mg/day to 0.66 mg/day of GLP-1 (e.g., in a human subject).

In some embodiments, body weight management includes inducing weight loss. In some embodiments, the body weight of the subject receiving the GLP-1 composition is less than 100%, less than 95%, less than 90%, less than 85%, less than 80%, less than 75% of the body weight of the subject who does not receive the GLP-1 composition.

### Slowing gastric emptying

In another aspect, provided herein is a method of slowing gastric emptying comprising administering to the subject a therapeutically effective amount of the pharmaceutical composition comprising GLP-1, the therapeutically effective amount is in the range from 0.11 mg/day to 0.66 mg/day of GLP-1 (e.g., in a human subject).

### Dosage of GLP-1

To achieve the same or a similar therapeutic effect for various conditions disclosed herein, the dosage of the GLP-1 composition is significantly lower than a pharmaceutical composition containing liraglutide. Additionally, the minimal dosage of GLP-1 to elicit a therapeutic effect in a female subject is significantly lower than the minimal dosage of GLP-1 to elicit the same or similar therapeutic effect in a male subject. In some embodiments, the minimal dosage of GLP-1 for a female subject is about 95%, about 90%, about 85%, about 80%, about 75%, about 70%, about 65%, about 60%, about 55%, about 50%, about 45%, about 40%, about 35%, about 30%, or about 25% of the minimal dosage of GLP-1 for a male subject to achieve the same or similar therapeutic effects. In general, a human dose is about 1/12 of the mouse dose, about 1/6 of the rat dose, or about 1/3 of the monkey dose.

The following examples are provided to better illustrate the embodiments. To the extent that specific materials are mentioned, it is merely for purposes of illustration.

### Example 1

This example demonstrates the effect of Beinaglutide injectable formulation on slowing stomach emptying. The Beinaglutideinjectable formulation contains Beinaglutide of SEQ ID NO: 1 at 4.2 mg (42,000 U) in 2.1 ml.

Fifty 8-week old Sprague-Dawley (SD) rats having a body weight of between 248 g and 308 g (male) or between 184 g and 218 g (female) were on adaptive feeding for 7 days, weight of each taken, and then divided into 5 groups: buffer only control, low dose of Beinaglutide (15 µg/kg), medium dose of Beinaglutide (30 µg/kg), high dose of Beinaglutide (60 µg/kg), and liraglutide control (270 µg/kg). Each group had 10 rats, half male and half female. The tails of the rats were labeled as follows:

| **Table 1** | | |
|---|---|---|
| **Group** | **Male Numbering** | **Female Numbering** |
| Buffer only control | 101-105 | 106-110 |
| low dose of Beinaglutide (15 µg/kg) | 201-205 | 206-210 |
| medium dose of Beinaglutide (30 µg/kg) | 301-305 | 306-310 |
| high dose of Beinaglutide (60 µg/kg) | 401-405 | 406-410 |
| liraglutide control (270 µg/kg) | 501-505 | 506-510 |

The dose selection is calculated based on human dose. The clinical human dose is estimated to be about 0.4 mg/injection, one injection per day, which corresponds to 36 µg/kg rat dose, calculated based on the body surface area. Accordingly, the rats were tested at a medium dose of 30 µg/kg, a low dose at 50% of the medium dose, and a high dose at 200% of the medium dose. The clinical human dose of liraglutide for treating obesity is 3.0 mg/injection, one injection per day, which corresponds to 270 µg/kg rat dose, calculated based on the body surface area.

Beinaglutide injectable formulation was provided by Shanghai Benemae Pharmaceutical Corporation (Benemae), which was a colorless, transparent liquid in 2.1 mL, containing 4.2 mg (42,000 U) of Beinaglutide. The Beinaglutide injectable formulation was stored in dark, at 2-8 °C. The buffer only control is a clinical placebo of the Beinaglutide injectable formulation, also provided by Benemae as a colorless, transparent liquid and stored at 2-8 °C. The positive control was liraglutide injectable formulation, which was stored in dark, sealed condition. The rats were raised and kept at the Laboratory Animal Center, Shanghai University of Traditional Chinese Medicine.

The injectable formulations were made and diluted at the time of experiment, as follows:

| **Table 2** | | | |
|---|---|---|---|
| **Injectable Formulation** | **Active Agent** | **Buffer** | **Dilution** |
| low dose of Beinaglutide (15 µg/kg) | 28.7 µl of Beinaglutide | 3971.3 µl | 140 fold |
| medium dose of Beinaglutide (30 µg/kg) | 57.4 µl of Beinaglutide | 3942.6 µl | 70 fold |
| high dose of Beinaglutide (60 µg/kg) | 114.8 µl of Beinaglutide | 3885.2 µl | 35 fold |
| liraglutide control (270 µg/kg) | 180 µl of liraglutide | 3820 µl | 22.2 fold |

The rats were fasted for 16 hours with only water provided. Before being divided into groups, the rats were weighed and weights were recorded (cage numbering and tail labeling). Based on the body weight, the rats were randomly divided into 5 groups, 10 in each group, half male and half female. After grouping, the rats were labeled as shown in Table 1.

The rats were administered with appropriate amounts of Beinaglutide or liraglutide based on their body weights by subcutaneous injection. Female rats were injected after male rats, in the order of their group numbers: 101, 201, 301, 401, 501, 102, 202, 302, 402, 502, etc. The formulations were injected to the rats by lifting the skin in the back, placing the needle of the syringe subcutaneously and injecting. When retrieving the needle, the site of injection was pressed with hand to avoid leakage of the formulation. The amount of injection is about 0.1 ml/100 g body weight.

Five minutes after subcutaneous injection, gavage was conducted using 5% carbon powder suspension (in 2% carboxymethylcellulose solution) at 1.5 ml/100 g body weight. After 15 minutes, the rats were sacrificed, and the abdominal cavity was cut open, and cardia and pylorus of the stomach were ligatured immediately. The stomach was wiped dry with filter paper and weighed for the gross weight of the stomach. Then the content of the stomach was removed and the stomach was weighed for the net weight. The respective distance from the pyloric sphincter to the front of carbon powder suspension and to the distal end of the large intestine was measured.
- propelling distance of carbon powder in the intestine (cm) = distance from pylorus to the proximal end of carbon powder;
- mass of carbon powder gavaged = concentration of carbon powder suspension X volume;
- propelling rate of carbon powder (%) = propelling distance of carbon powder in the intestine (cm)/entire length of intestine (cm) X 100%;
- rate of gastric remains (%) = (gross weight of stomach - net weight of stomach)/mass of carbon powder gavaged X 100%.

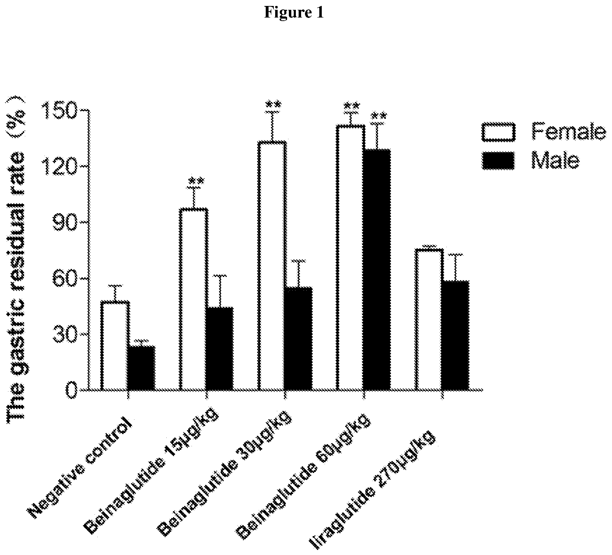

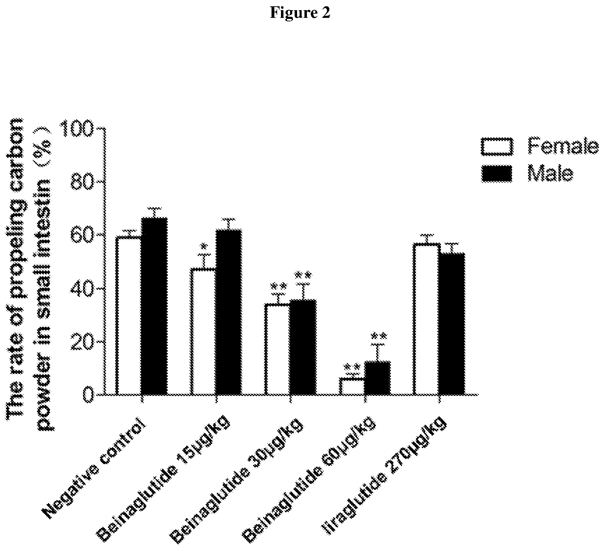

As shown in Tables 3 and 4 and Figures 1 and 2, the group of the male rats administered with high dose of Beinaglutide, and the groups of the female rats administered with low dose, medium dose, and high dose of Beinaglutide exhibited a higher rate gastric remains than the buffer only control groups, the difference between the Beinaglutide group and the control group was statistically significant. The groups of the male rats administered with medium dose and high dose of Beinaglutide, and the groups of the female rats administered with low dose, medium dose, and high dose of Beinaglutide exhibited a lower propelling rate of carbon powder than the buffer only control groups, the difference between the Beinaglutide group and the control group was statistically significant. Although liraglutide showed certain level of inhibition on stomach emptying and propelling rate of carbon powder, the difference between the liraglutide group and control group was not statistically significant. Accordingly, Beinaglutide but not liraglutide can effectively slow stomach emptying in rats.

| Table 3 Stomach Emptying of Male Rats (*X̅*±SE) | | | | | |
|---|---|---|---|---|---|
| Groups | Gross Weight of Stomach (g) | % of Gastric Remains (%) | Propelling Distance of Carbon Powder (cm) | Entire Length of Intestine (cm) | Propelling Rate of Carbon Powder (%) |
| Buffer only control | 2.33±0.06 | 23.37±3.15 | 65.54±4.46 | 98.68±1.55 | 66.31±3.68 |
| Low dose of Beinaglutide | 2.86±0.52 | 44.14±17.18 | 64.22±3.84 | 104.18±1.76 | 61.80±4.14 |
| Medium dose of Beinaglutide | 3.08±0.48 | 54.92±14.32 | 34.40±5.91^{**} | 97.52±3.57 | 35.46±6.15^{**} |
| High dose of Beinaglutide | 5.36±0.52^{**} | 128.70±14.23^{**} | 11.90±6.38^{**} | 101.32±3.77 | 12.28±6.69^{**} |
| Liraglutide control | 3.14±0.30 | 58.29±14.55 | 54.66±4.35 | 103.20±3.05 | 52.94±3.82 |

| | | | | | |
|---|---|---|---|---|---|
| Note: Comparing to buffer only control, * *P*<0.05, ** *P*<0.01. | | | | | |

| Table 4 Stomach Emptying of Female Rats (*X̅*±SE) | | | | | |
|---|---|---|---|---|---|
| Groups | Gross Weight of Stomach (g) | % of Gastric Remains (%) | Propelling Distance of Carbon Powder (cm) | Entire Length of Intestine (cm) | Propelling Rate of Carbon Powder (%) |
| Buffer only control | 2.14±0.16 | 47.24±8.79 | 53.86±4.01 | 90.96±3.16 | 58.96±2.58 |
| Low dose of Beinaglutide | 3.10±0.22^{*} | 96.81±11.61^{**} | 41.96±5.30^{*} | 88.98±1.44 | 46.97±5.54^{*} |
| Medium | 3.78±0.35^{**} | 132.66±16.34^{**} | 30.58±3.66^{**} | 90.26±1.28 | 33.88±3.98^{**} |
| dose of Beinaglutide | | | | | |
| High dose of Beinaglutide | 3.90±0.15^{**} | 141.53±7.18^{**} | 5.40±1.84^{**} | 89.70±2.59 | 5.94±1.99^{**} |
| Liraglutide control | 2.64±0.06 | 75.13±2.11 | 51.46±2.23 | 91.94±3.13 | 56.38±3.56 |

| | | | | | |
|---|---|---|---|---|---|
| Note: Comparing to buffer only control, * *P*<0.05, ** *P*<0.01. | | | | | |

### Example 2

This example demonstrates the activation effect of Beinaglutide on GLP-1 receptor signaling pathway.

GLP-1 receptor belongs to the family of G-protein coupled receptors and has seven transmembrane domains. The effects of Beinaglutide and the positive control GLP-1(7-36)-NH₂ on GLP-1 receptor were analyzed using CHO-K1 cells stably expressing GLP-1 receptor via a fluorescence detection of intracellular calcium levels. Beinaglutide induced intracellular calcium flow in the CHO-K1 cells in a concentration-dependent manner. Beinaglutide and the positive control GLP-1(7-36)-NH₂ exhibited similar EC50, at 118 ± 48 and 141 ± 18 nM, respectively. Both Beinaglutide and GLP-1(7-36)-NH₂ fully demonstrated activation effects on the GLP-1 receptor signaling pathway.

### Example 3

This example demonstrates the effect of Beinaglutide on blood sugar levels in animals in normal and diabetic animal models.

Beinaglutide was able to lower glucose-induced high blood sugar in mice and rabbits with normal glycemic index (GI), as well as to reduce the increase of blood sugar induced by alloxan and streptozotocin in mice and rats with normal GI, demonstrating its effects on lowering blood sugar. Animals having increased blood sugar had a normal GI, and when the blood sugar decreased, insulin level increased significantly and correlated with the decrease of blood sugar. However, in animals having a normal level of blood sugar, Beinaglutide neither decreased blood sugar nor increased blood insulin level, indicating that Beinaglutide promoted insulin secretion was dependent on the concentration of glucose.

### Example 4

This example demonstrates the effect of Beinaglutide on reducing body weight in diet-induced obesity cynomolgus monkeys.

Beinaglutide injectable formulation was subcutaneously administered to diet-induced obesity cynomolgus monkeys twice or three times a day, for a period of three weeks to evaluate the effects on decreasing appetite and body weight loss as well as the continuous effects and reversibility once the treatment was terminated.

Two male (10.5-11.5 kg) and two female (6.0-6.5 kg) obesity cynomolgus monkeys between 14-19 years old and having a BMI greater than 40 kg/m² were used in this study. As shown in Figure 3, this study included blank control (buffer only) and subject own control, multiple incremental doses, and cross-administration. The monkeys are divided into two groups based on age, sex, body weight and BMI, each group having one male and one female.

| **Table 5** | | | | |
|---|---|---|---|---|
| | **Group 1** | | **Group 2** | |
| Sex | Male | Female | Male | Female |
| Age | 18.6 | 14.7 | 18.8 | 18.0 |
| Body Weight (kg) | 11.06 | 6.02 | 10.52 | 6.18 |
| Crown-Rump Height (m) | 0.47 | 0.38 | 0.45 | 0.39 |
| BMI | 50 | 42 | 52 | 41 |
| DEXA Lean Mass (kg) | 7.53 | 4.11 | 6.88 | 3.17 |
| DEXA Body Fat (kg) | 3.30 | 1.93 | 3.49 | 2.97 |
| DEXA Body Fat Percentage (%) | 30.4 | 31.9 | 33.7 | 48.3 |

The study included three periods: baseline period (the first week), treatment period (the second week through the seventh week), and recovering period (the eighth week). After the monkeys adapted to the meal time for at least 30 days, the body weight, BMI, and body composition of each animal was determined at day 7 of the first baseline period. These indexes were used as the baseline for evaluating the efficacy of Beinaglutide.

During the treatment period (second to seventh week), Group 1 monkeys were subcutaneously administered with blank control three times a day during week 2 through week 4, with Beinaglutide injectable formulation twice a day during week 5 through week 7. Group 2 monkeys were subcutaneously administered with Beinaglutide injectable formulation three times a day during week 2 through week 4, with blank control twice a day during week 5 through week 7. To improve tolerance of Beinaglutide and to determine the maximum tolerance dose for Beinaglutide, the dose of Beinaglutide was increased gradually during the study. Group 2 animals were administered three times a day at a dose of 10 µg/kg each time during week 2, three times a day at a dose of 20 µg/kg each time during week 3, and three times a day at a dose of 40 µg/kg each time during the first three days of week 4. Due to severe anorexia observed, the dose was reduced to 20 µg/kg each time during the remaining 4 days of week 4, shown in Table 6.

| **Table 6** | | | | | | |
|---|---|---|---|---|---|---|
| Group No. | Dose (µg/kg each time) | | | | | |
| | Week 2 | Week 3 | Week 4 | Week 5 | Week 6 | Week 7 |
| 1 | Blank control | | | 10 | 20 | 20 |
| 2 | 10 | 20 | 40/20 | Blank control | | |

The dose for Group 1 animals during week 5 through week 7 was determined based on the dose given to Group 2 animals during week 2 through week 4. Group 1 animals were administered twice a day at a dose of 10 µg/kg each time during week 5, twice a day at a dose of 20 µg/kg each time during week 6, and twice a day at a dose of 20 µg/kg each time during week 7 (same dose as week 6). During the 6-week treatment period, the body weight and crown-rump height of each animal were measured at the end of each week. The body fat content was measured at week 4 and week 7. No administration was given during week 8.

During the eight weeks of this study, the amount of the food intake of each meal for each animal was recorded. The effects of Beinaglutide of Group 1 on affecting the food intake, body weight and BMI were evaluated by comparing to the baseline levels during week 4 and week 7. The effects of Beinaglutide of Group 2 on affecting the food intake, body weight and BMI were evaluated by comparing to the baseline levels during week 1 and week 4.

Beinaglutide was diluted 30 minutes prior to injection from 2 mg/ml to 0.2, 0.4, or 0.8 mg/ml for injection in a volume of 0.05 ml/kg. The animals were given three meals a day, including monkey feed in the morning (around 8:30) and in the afternoon (around 16:00) and fruits in between (around 14:00). Each animal was given 150 g monkey feed. After half an hour, if all monkey feed was consumed, the animal was given an extra 50 g of monkey feed. The animals were given about one hour to finish each meal and the leftover was weighed and recorded to calculate the food intake. Each animal was provided with the same calories of fruits, based on Table 7.

| **Table 7** | | | | | | |
|---|---|---|---|---|---|---|
| Fruits | Apple | Pear | Papaya | Watermelon | Tomato | Carrot |
| Calories per 100 g | 52 kCal | 43 kCal | 27 kCal | 34 kCal | 19 kCal | 37 kCal |
| Fruits for each meal | 66 g | 80 g | 127 g | 101 g | 180 g | 92 g |
| Total Calories | 34 kCal | 34 kCal | 34 kCal | 34 kCal | 34 kCal | 34 kCal |

The body weight was measured once a week, and crown-rump height was measured at the end of week 1, week 4, and week 7 when Dual-Energy X-ray Absorptiometry (DEXA) scan was taken. The animals were anesthetized by intramuscular administration of 0.02 ml/kg Shumianning. While resting on one side, the animals were measured from the top of the head to the bottom of tail as the crown-rump height. BMI was calculated using the formula: BMI = body weight (kg)/crown-rump height (m)^2. DEXA scan was conducted while the animals were anesthetized and body compositions were analyzed based on DEXA scan.

During the study, the animals were observed for at least three times a day before and during feeding for any abnormal behavior, or signs of pain or tension. After being administered with Beinaglutide or blank control, the animals were observed for any of the following symptoms, including loss of appetite, sleepiness, diarrhea, constipation, indifference, vomiting, inflammation or any other conditions around the injecting area. If symptoms of low blood sugar, such as shivering or coma, were observed, the blood glucose test was given to the animals to confirm low blood sugar and the animals were given conservative treatment.

### Food Intake

The daily food intake for each animal was monitored during the entire study period. As shown in Figure 4A, Group 1 animals had the same food intake during week 2 through week 4 when administered with blank control as the food intake during week 1, and significantly lower food intake during week 5 through week 7 when administered with Beinaglutide. The food intake during week 8 was recovered during week 8, comparable to week 1. As shown in Figure 4B, Group 2 animals had significantly lower food intake during week 2 through week 4 when administered with Beinaglutide, compared to food intake during week 1. The appetite started to recover during week 5 and recovered to baseline after week 7 when administration was completed.

The calories intake for each animal was calculated based on the food intake and the values before, during and after treatment with Beinaglutide were compared, as shown in Figure 4C. The weekly food/calories intake for each animal during Beinaglutide treatment period was significantly decreased compared to the intake before treatment (p < 0.05).

Accordingly, two or three subcutaneous injections of Beinaglutide per day significantly reduced food intake in both male and female obesity monkeys. In fact, Group 2 animals showed severe signs of anorexia when administered with a dose of 40 µg/kg Beinaglutide in the first three days of week 4, and therefore, the dose had to decrease to 20 µg/kg during the remaining days of week 4. It was estimated that the maximum tolerance dose of Beinaglutide for diet induced obesity cynomolgus monkeys was three times a day, 20-40 µg/kg each time.

### Body weight and BMI

During the entire study period, each monkey was weighed on a weekly basis and all body weigh information was recorded in Table 8.

| **Table 8** | | | | | | | | | |
|---|---|---|---|---|---|---|---|---|---|
| | Animal | Week 1 | Week 2 | Week 3 | Week 4 | Week 5 | Week 6 | Week 7 | Week 8 |
| Body Weight (kg) | M1 | 11.06 | 11.08 | 11.18 | 11.20 | 10.90 | 10.44 | 10.26 | 10.48 |
| | F1 | 6.02 | 5.94 | 6.00 | 5.96 | 5.92 | 5.94 | 5.84 | 5.88 |
| | M2 | 10.52 | 10.30 | 10.00 | 9.70 | 9.62 | 9.66 | 9.54 | 9.64 |
| | F2 | 6.18 | 5.96 | 5.76 | 5.56 | 5.54 | 5.74 | 5.72 | 5.90 |
| Crown to Rump Height (m) | M1 | 0.49 | - | - | 0.48 | - | - | 0.47 | - |
| | F1 | 0.38 | - | - | 0.39 | - | - | 0.38 | - |
| | M2 | 0.49 | - | - | 0.47 | - | - | 0.45 | - |
| | F2 | 0.38 | - | - | 0.37 | - | - | 0.39 | - |
| BMI (kg/m²) | M1 | 50 | 50 | 51 | 51 | 49 | 47 | 46 | 47 |
| | F1 | 42 | 41 | 42 | 41 | 39 | 39 | 38 | 39 |
| | M2 | 52 | 51 | 49 | 48 | 44 | 44 | 43 | 44 |
| | F2 | 41 | 39 | 38 | 37 | 40 | 42 | 42 | 43 |

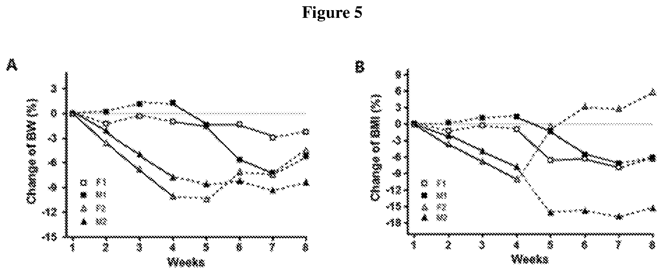

The male and female animals in Group 1 did not show any substantial change in body weight during week 2 through week 4 while blank control was administered; in contrast, the monkeys in Group 2 showed continuous weight loss since Beinaglutide was administered, as shown in Figure 5A. Additionally, Group 1 animals showed body weight loss during week 5 through week 7 when Beinaglutide was administered. The female monkey in Group 2 showed gradual weight gain when switched to blank control in week 5; however, the male monkey in Group 2 did not have any substantial weight gain when the Beinaglutide administrated ended. As shown in Figure 5B, the body weight of each animal was compared before and after Beinaglutide treatment. The animals showed significant weight loss after three-week Beinaglutide treatment when compared to the body weight of the baseline period (p < 0.05).

Crown to rump height of each animal was measured by the end of week 1, week 4, and week 7, and BMI was calculated based on the body weight and crown to rump height. BMI decreased in the two groups of animals after three weeks of Beinaglutide treatment.

### Analysis of body composition

DEXA scan was performed on each animal at week 1, week 4, and the end of week 7 and information about body composition is shown in Table 9.

| **Table 9** | | | | | | | | | | | | |
|---|---|---|---|---|---|---|---|---|---|---|---|---|
| | Group 1 | | | | | | Group 2 | | | | | |
| | M1 | | | F1 | | | M2 | | | F2 | | |
| | Lean Mas s (kg) | Fat Con tent (kg) | Body Fat Percen tage (%) | Lean Mas s (kg) | Fat Con tent (kg) | Body Fat Percen tage (%) | Lean Mas s (kg) | Fat Con tent (kg) | Body Fat Percen tage (%) | Lean Mas s (kg) | Fat Con tent (kg) | Body Fat Percen tage (%) |
| Baseline Week 1 | 7.53 | 3.30 | 29.8 | 4.11 | 1.93 | 32.1 | 6.88 | 3.49 | 33.2 | 3.17 | 2.97 | 48.1 |
| Week 4 | 7.35 | 3.66 | 32.7 | 4.09 | 1.98 | 33.2 | 6.59 | 2.88 | 29.7 | 2.89 | 2.71 | 48.7 |
| Week 7 | 6.92 | 3.15 | 30.7 | 3.98 | 1.81 | 31.0 | 6.75 | 2.7 | 28.3 | 3.28 | 2.53 | 44.2 |

At the end of week 4, Group 1 animals which were administered with blank control had a slight increase in body fat content compared to the baseline level, however, Group 2 animals which were administered with Beinaglutide had a significant decrease in body fat content. At the end of week 7, Group 1 animals which were administered with Beinaglutide also had a decrease in body fat content compared to the baseline level. The body fat percentage was calculated based on the percentage of body fat in total body weight.

### Example 5

In trial 5A, Beinaglutide (four dose groups of 0 mg, 0.1 mg, 0.15 mg, and 0.2 mg, three times a day, 0 mg, 0.1 mg, 0.15 mg or 0.2 mg each time) and/or glipizide (glipizide controlled release tablets (Rui Yining), 5 mg once a day orally) was administered to type 2 diabetic patients (96 patients) with poor glycemic control of sulfonylureas (e.g., glipizide). To observe the changes in body weight and BMI, and adverse reactions such as gastrointestinal reactions in the subjects treated with Beinaglutide and glipizide alone or in combination at different doses.

The drug of choice for the treatment of diabetes is metformin. In trial 5B, Beinaglutide (0 mg, 0.1 mg, 0.15 mg, and 0.2 mg in four dose groups, three times a day, 0 mg, 0.1 mg, 0.15 mg or 0.2 mg each time) and metformin (oral monotherapy, therapeutic dose ≥1,000 mg/day, the dose has been stabilized for at least 3 months) was administered to type 2 diabetic patients (240 patients) with poor metformin control alone. Systematic observation of the effects of Beinaglutide and metformin administered alone or in combination was conducted respectively.

The results of trials 5A and 5B showed that the effect of Beinaglutide was fast after injection, and the peak time was 20 minutes after administration. Despite its short duration of action (half-life of approximately 15 minutes), when combined with glipizide or metformin, it showed a better therapeutic effect than glipizide or metformin alone. Adverse events in Beinaglutide clinical trials were mainly mild to moderate nausea and vomiting, most of which can be tolerated by the subjects and relieved during medication.

Trial 5C was based on trial 5B, increasing the number of patients with type 2 diabetes, designing an expanded multicenter clinical trial protocol to further evaluate the effectiveness and safety of the combination of Beinaglutide (0.2 mg dose, three times a day, 0.2 mg each time) and oral hypoglycemic agents (metformin).

Table 10 (Trial 5A, in combination with glipizide), Table 11 (Trial 5B, in combination with metformin) and Table 12 (Trial 5C, in combination with metformin) showed 12-week BMI changes in patients with type 2 diabetes .

**Table 10: BMI (kg/m²) treatment data for each group of Trial 5A**

| Groups | Full analysis set (FAS) | | | | |
|---|---|---|---|---|---|
| (Beinaglutide doses) | Before treatment | Treatment for 2 weeks | Treatment for 4 weeks | Treatment for 8 weeks | Treatment for 12 weeks |
| 0 mg group | 25.52±4.06 (n=24) | 25.33±4.35 | 25.40±4.09 | 25.45±3.94 | 25.32±3.94 |
| 0.1 mg group | 25.51±2.59 (n=24) | 25.53±2.63 | 25.40±2.64 | **25.25±2.57*** | **25.20±2.60*** |
| 0.15 mg group | 25.66±2.64 (n=24) | 25.63±2.57 | 25.45±2.63 | **25.19±2.48*** | **25.11±2.60**** |
| 0.2 mg group | 26.71±4.17 (n=23) | 26.44±4.14 | 26.52±3.95 | **26.09±4.04**** | **25.92±3.95**^{#}** |

| | | | | | |
|---|---|---|---|---|---|
| Note: ①Group comparison *P<0.05, **P<0.001; ②Reduced value after treatment compared with 0mg group ^{#}P<0.05, ^{##}P<0.01 | | | | | |

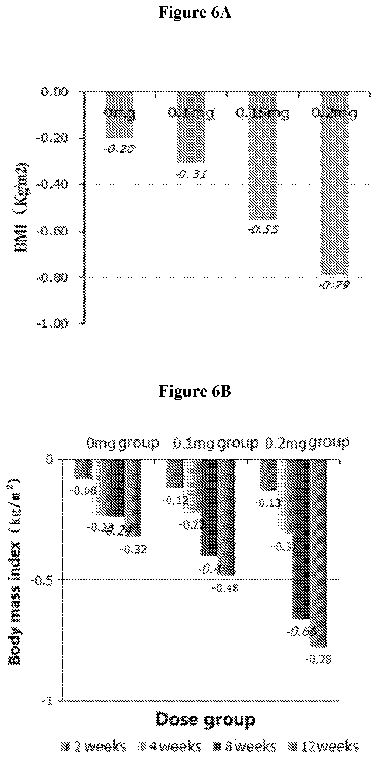

The body mass index of the three Beinaglutide groups (0.1 mg, 0.15 mg, and 0.2 mg) showed statistically significant (P < 0.05) body weight loss at weeks 8 and 12 of treatment. In the 0.2 mg group, the body mass index (kg/m2) at the 12th week of treatment decreased by 0.79±0.79 (P<0.001), and the decrease was significantly different from the 0 mg dose group (P<0.05) (Figure 6A).

**Table 11: BMI (kg/m²) treatment data from Trial 5B**

| Group (Beinaglutid e doses) | Full analysis set (FAS) | | | | |
|---|---|---|---|---|---|
| | Day 1 of treatment | Treatment for 2 weeks | Treatment for 4 weeks | Treatment for 8 weeks | Treatment for 12 weeks |
| 0 mg group | 25.64±3.20 (n=70) | 25.51±3.22 | 25.36±3.16** | 25.35±2.99** | 25.29±3.13** |
| 0.1 mg group | 26.24±3.31 (n=73) | 26.12±3.29* | 26.02±3.33** | 25.84±3.25** | 25.76±3.21** |
| 0.2 mg group | 25.14±3.10 (n=70) | 25.03±3.21* | 24.85±3.25** | 24.54±3.33** | 24.43±3.42** |

| | | | | | |
|---|---|---|---|---|---|
| Note: ①Group comparison *P<0.05, **P<0.01 | | | | | |

The changes in body mass index of the three groups in the trial 5B were basically consistent with the changes in body weight. A 12-week comparison between groups showed that the body mass index of the 0.2 mg group reduced most significantly, with significant differences between the 0 mg and 0.1 mg groups (P = 0.0000 and P = 0.0020). There was no significant difference between the 0 mg and 0.1 mg groups (P=0.2317) (Figure 6B).

As shown in Tables 10-11, when Beinaglutide was used in combination with glipizide or metformin, the BMI of the subject was significantly reduced in a dose-dependent manner.

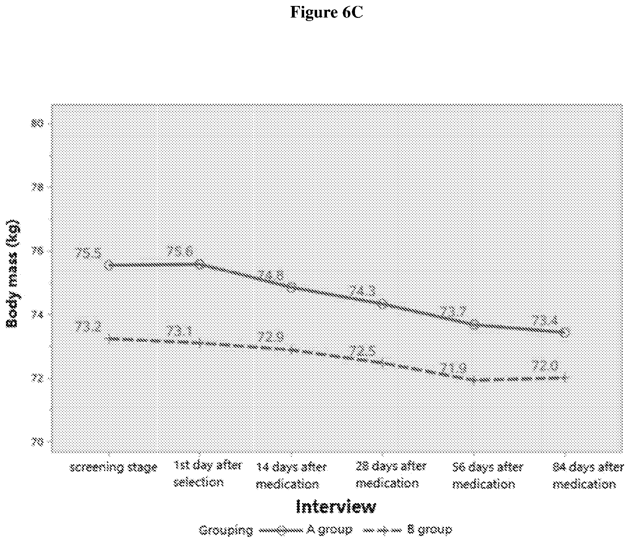

Data from trial 5C were analyzed according to BMI criteria for overweight subjects (27.9 ≥ BMI ≥ 24.0 kg/m²) and obese patients (BMI ≥ 28.0 kg/m²) (12 weeks of treatment (84 days)). Tables 12A, 12B and 13 show the effects of Beinaglutide on overweight and obese subjects, respectively (Figure 6C).

| **Table 12A:** | | |
|---|---|---|
| Trial 5C (Beinaglutide doses) | 0 mg | 0.2 mg |
| Baseline BMI (kg/m²) | 25.59 | 25.81 |
| Week12 BMI (kg/m²) | 25.18 | 25.09 |
| BMI Change | -0.40 | -0.72 |

**Table 12B: Diachronic changes in body weight (kg) (ITT) of overweight and obese patients during the trial (test 5C data)**

| Classification | | Test | Control | Total |
|---|---|---|---|---|
| Changes from baseline to 84 days after medication | | | | |
| | Number of subjects (missing) | 248(4) | 77(1) | 325(5) |
| | Mean ± standard deviation | -2.22±2.55 | -1.24±1.96 | -1.99±2.45 |
| | median | -2.00 | -1.00 | -1.70 |
| | Q1~Q3 | -3.50~-0.50 | -2.50~0.00 | -3.00~0.00 |
| | Minimum to maximum | -13.7~3.20 | -7.30~3.00 | -13.7~3.20 |
| | 95%CI | -2.54~-1.91 | -1.69~-0.80 | -2.26~-1.72 |
| | In-group pairwise test p-value | <0.0001 | <0.0001 | <0.0001 |

| 84 days decline rate from baseline after medication 1 | | | | |
|---|---|---|---|---|
| | Weight loss≥3% | 100(40.32) | 21 (27.27) | 121 (37.23) |
| | Weight loss<3% | 148(59.68) | 56(72.73) | 204(62.77) |

| 84 days decline rate from baseline after medication 2 | | | | |
|---|---|---|---|---|
| | Weight loss≥5% | 52(20.97) | 8(10.39) | 60(18.46) |
| | Weight loss <5% | 196(79.03) | 69(89.61) | 265(81.54) |

**Table 13: Covariance analysis of body weight versus baseline between groups (Trial 5C data)**

| | | Corrected mean | Standard error | 95% Cl lower limit | 95% Cl upper limit | P Value |
|---|---|---|---|---|---|---|
| 84 days after medication | | | | | | |
| | Test group | -2.19 | 0.149 | -2.48 | -1.89 | |
| | Control group | -1.36 | 0.267 | -1.89 | -0.84 | |
| | Test-Control group | -0.82 | | -1.43 | -0.22 | 0.0075 |

| | | | | | | |
|---|---|---|---|---|---|---|
| Note: Covariance analysis was used for comparison between groups. The dependent variable was the change in body weight relative to baseline, and the covariate was baseline. | | | | | | |

Body weight change from baseline = post-treatment - baseline. Data from 5C test data.

As shown in Tables 12 and 13, after 12 weeks of treatment, the proportion of subjects in the Beinaglutide + metformin group with the weight loss from the baseline ≥ 3% was 40.32%, and the proportion of subjects with weight loss ≥ 5% was 20.97. %, the decline rate of the control group (metformin alone group) was 27.27% and 10.39%, respectively. Paired T test and covariance analysis between groups were performed for the changes from baseline after treatment. All the p values were <0.05, and the differences were statistically significant. Statistical analysis showed that the Beinaglutide + metformin treatment group was superior to the control group in weight loss.

### Example 6

This example demonstrates the effects of various dosages of Beinaglutide injectable formulation on slowing stomach emptying in male and female rats. The Beinaglutide injectable formulation contains Beinaglutide of SEQ ID NO: 1 at 4.2 mg (42000 U) in 2.1 ml.

One hundred and twelve 8-week old Sprague-Dawley (SD) rats having a body weight of between 240 g and 287 g (male) or between 194 g and 227 g (female) were divided into 7 groups: Group 1 (buffer only control), Group 2 (Beinaglutide 10 µg/kg), Group 3 (Beinaglutide 12.5 µg/kg), Group 4 (Beinaglutide 15 µg/kg), Group 5 (Beinaglutide 30 µg/kg), Group 6 (Beinaglutide 45 µg/kg), and Group 7 (Beinaglutide 60 µg/kg). Various dosages of Beinaglutide solutions were prepared from the Beinaglutide injectable formulation, kept at room temperature for at least 3 hours before injecting the animals. The animals were fasted 29-33 hours before injection, and the animals were not given any water 5-7 hours before injection. Injections were made by subcutaneous administration to the neck, at 0.1 ml/100 g body weight. The experiment was carried as described in Example 1.

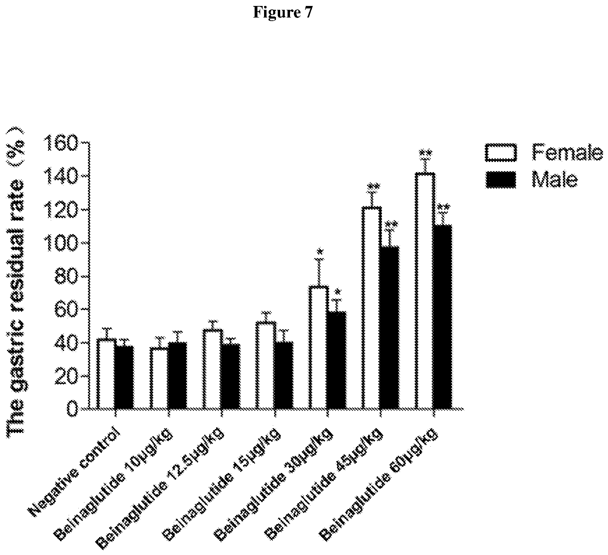

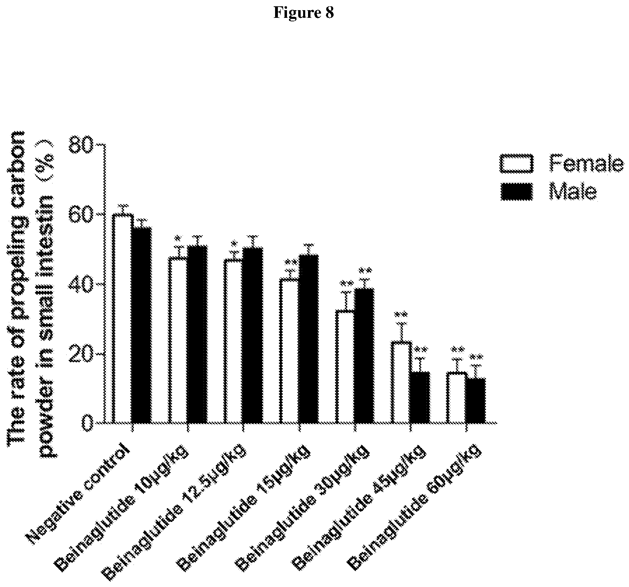

As shown in Tables 14 and 15 and Figures 7 and 8, both male and female rats received 30 µg/kg, 45 µg/kg, or 60 µg/kg of Beinaglutide exhibited a rate of remains in the stomach higher than the blank only control. The male rats received 30 µg/kg, 45 µg/kg, or 60 µg/kg of Beinaglutide, as well as the female rats received 10 µg/kg, 12.5 µg/kg, 15 µg/kg, 30 µg/kg, 45 µg/kg, or 60 µg/kg of Beinaglutide, exhibited a propelling rate of carbon powder lower than the blank only control. Accordingly, the data demonstrated that at a dosage corresponding to the human clinical dosage, injection of Beinaglutide significantly inhibited stomach emptying. The minimal effective dosage for male rats and female rats are 30 µg/kg and 10 µg/kg, respectively, corresponding to human doses of 0.33 mg/day and 0.11 mg/day, respectively.

| Table 14 Stomach Emptying of Male Rats (*X̅*±SE) | | | | | |
|---|---|---|---|---|---|
| Groups | Gross Weight of Stomach (g) | % of Gastric Remains (%) | Propelling Distance of Carbon Powder (cm) | Entire Length of Intestine (cm) | Propelling Rate of Carbon Powder (%) |
| Buffer only control | 2.40±0.14 | 37.68±4.15 | 60.73±1.64 | 108.90±2.10 | 55.98±2.30 |
| Beinaglutide (10 µg/kg) | 2.39±0.19 | 39.93±6.46 | 55.20±2.69 | 108.83±2.57 | 50.91±2.84 |
| Beinaglutide (12.5 µg/kg) | 2.36±0.09 | 38.98±3.50 | 54.96±3.96 | 108.98±2.28 | 50.36±3.34 |
| Beinaglutide (15 µg/kg) | 2.42±0.20 | 40.22±7.06 | 52.27±3.75 | 108.15±2.21 | 48.22±3.05 |
| Beinaglutide (30 µg/kg) | 2.92±0.23 | 58.23±7.38^{*} | 42.82±2.81^{**} | 111.63±1.57 | 38.55±2.81^{**} |
| Beinaglutide (45 µg/kg) | 3.90±0.27^{**} | 97.66±9.95^{**} | 15.73±4.30^{**} | 108.99±2.37 | 14.67±4.13^{**} |
| Beinaglutide (60 µg/kg) | 4.21±0.19 | 110.28±7.79^{**} | 14.64±4.32^{**} | 111.16±1.49 | 12.91±3.68^{**} |

| | | | | | |
|---|---|---|---|---|---|
| Note: Comparing to buffer only control, * *P*<0.05, ** *P*<0.01. | | | | | |

| Table 15 Stomach Emptying of Female Rats (*X̅*+SE) | | | | | |
|---|---|---|---|---|---|
| Groups | Gross Weight of Stomach (g) | % of Gastric Remains (%) | Propelling Distance of Carbon Powder (cm) | Entire Length of Intestine (cm) | Propelling Rate of Carbon Powder (%) |
| Buffer only control | 1.98±0.13 | 41.63±6.82 | 63.79±2.35 | 107.16±1.73 | 59.76±2.70 |
| Beinaglutide (10 µg/kg) | 1.86±0.14 | 36.42±6.50 | 53.01±3.12^{*} | 106.51±1.64 | 49.92±2.97^{*} |
| Beinaglutide (12.5 µg/kg) | 2.03±0.10 | 47.54±5.22 | 49.50±2.54^{*} | 105.72±0.42 | 46.83± 2.41^{*} |
| Beinaglutide (15 µg/kg) | 2.18±0.11 | 51.86±6.07 | 44.50±2.97^{**} | 107.88±2.05 | 41.26±2.63 |
| Beinaglutide (30 µg/kg) | 2.59±0.38^{*} | 73.35±16.68^{*} | 33.96±5.58^{**} | 106.90±1.36 | 32.15±5.54^{**} |
| Beinaglutide (45 µg/kg) | 3.55±0.21^{**} | 120.95+9.48^{**} | 25.06±5.91^{**} | 107.48±1.06 | 23.18±5.46^{**} |
| Beinaglutide (60 µg/kg) | 3.90±0.20^{**} | 141.28±8.90^{**} | 15.71±4.58^{**} | 108.24±1.42 | 14.36±4.08^{**} |

| | | | | | |
|---|---|---|---|---|---|
| Note: Comparing to buffer only control, * *P*<0.05, ** *P*<0.01. | | | | | |

### Example7

This example demonstrates the effects of various dosages of Beinaglutide injectable formulation on slowing stomach emptying in male diet induced obesity (DIO) mice. Except that a different animal model was used, the experiment was performed in the same way as described in Example 6.

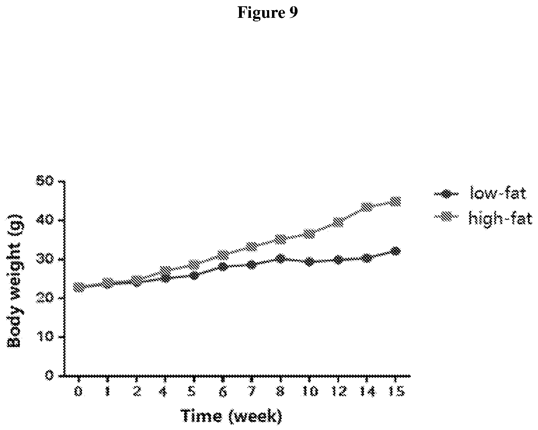

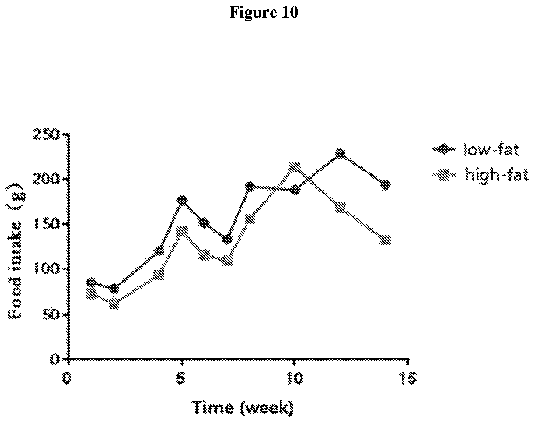

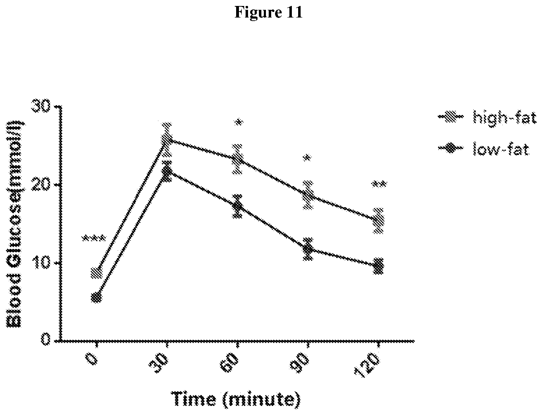

Fifty-six C57BL/6J mice between 4-6 weeks old were fed with high-fat diet (60% fat in calorie) and low-fat diet (10% fat in calorie), respectively, for 15 weeks until the body weight of the mice fed with high-fat diet significantly increased to 39.2-47.9 g. Figure 9 and Figure 10 show the body weight change and food intake of the mice on high-fat diet and low-fat diet over the period of 15 weeks. As the end of special diet period, successful induction of insulin resistance and obesity was confirmed by the intraperitoneal glucose tolerance test (IPGTT) shown in Figure 11.

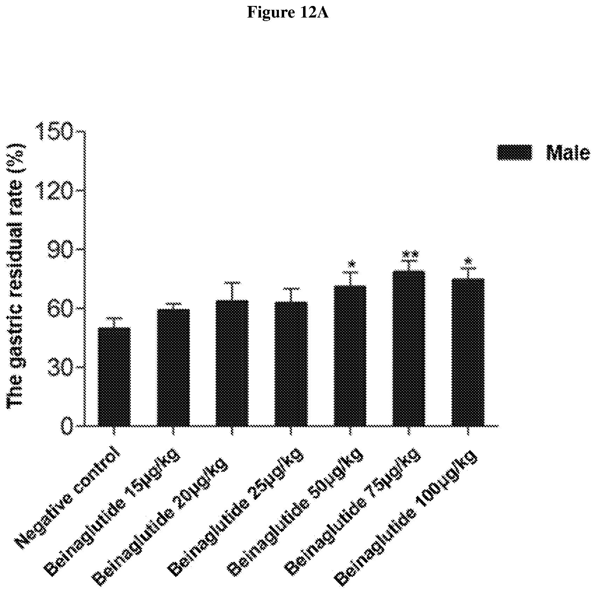

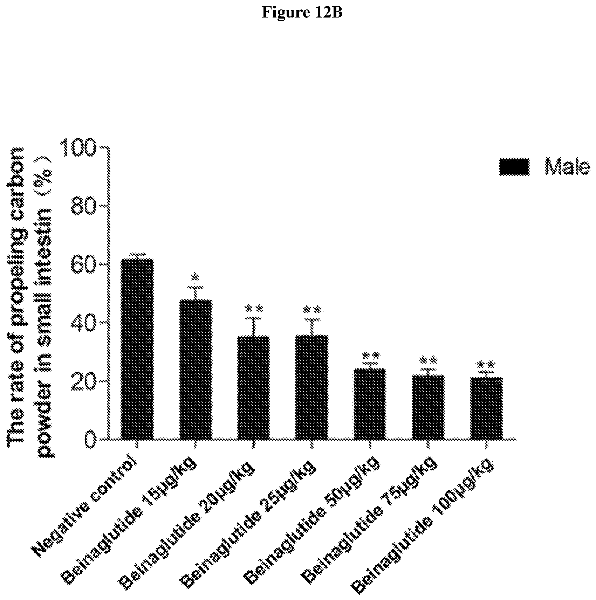

As shown in Table 16 and Figures12A and 12B, the male DIO mice received 50 µg/kg or more Beinaglutide exhibited a rate of remains in the stomach significantly higher than the buffer only control. The male DIO mice received 15 µg/kg or more Beinaglutide exhibited a propelling rate of carbon powder significantly lower than the blank control. Accordingly, the data demonstrated that at a dosage corresponding to the human clinical dosage, injection of Beinaglutide significantly inhibited stomach emptying in DIO mice. The effective dose can be as low as 15 µg/kg, corresponding to a human dose of 0.12 mg/day.

| Table 16 Stomach Emptying of DIO Mice (*X̅*±SE) | | | | | |
|---|---|---|---|---|---|
| Groups | Gross Weight of Stomach (g) | % of Gastric Remains (%) | Propelling Distance of Carbon Powder (cm) | Entire Length of Intestine (cm) | Propelling Rate of Carbon Powder (%) |
| Buffer only control | 0.46±0.03 | 49.50±5.37 | 26.00±1.07 | 42.40±0.34 | 61.28±2.25 |
| Beinaglutide (15 µg/kg ) | 0.49±0.02 | 58.92±3.39 | 20.50±1.89 | 43.57±0.87 | 47.34±4.63 |
| Beinaglutide (20 µg/kg) | 0.50±0.04 | 63.46±9.45 | 14.21±2.59 | 41.36±1.15 | 34.86±6.72 |
| Beinaglutide (25 µg/kg) | 0.53±0.03 | 62.69±7.26 | 15.00±2.45 | 42.14±1.23 | 35.25±5.72 |
| Beinaglutide (50 µg/kg) | 0.55±0.02 | 70.86±7.41 | 10.00±1.04 | 42.00±0.53 | 23.80±2.41 |
| Beinaglutide (75 µg/kg) | 0.57±0.03 | 78.34±5.75 | 9.29±1.23 | 42.86±0.77 | 21.51±2.63 |
| Beinaglutide (100 µg/kg) | 0.56±0.02 | 74.37±6.06 | 9.00±1.12 | 42.71±0.89 | 20.87±2.19 |

## Claims

1. A composition comprising a GLP-1 peptide for use in a method of treatment of obesity, wherein the method comprises administering to a human subject suffering from obesity a therapeutically effective amount of a composition comprising a GLP-1 peptide, wherein the GLP-1 peptide is GLP-1 (7-36), wherein the therapeutically effective amount of the GLP-1 peptide is from 0.11 mg/day to 0.66 mg/day, and wherein the composition is administered by two or three injections per day.

2. The composition for use of claim 1, wherein the subject is a male subject.

3. The composition for use of any one of the preceding claims, wherein the therapeutically effective amount of the GLP-1 peptide is from 0.33 mg/day to 0.66 mg/day.

4. The composition for use of claim 1, wherein the subject is a female subject.

5. The composition for use of any one of the preceding claims, wherein the composition is administered by parenteral injection, intravenous injection or subcutaneous injection.

6. A cosmetic method of managing body weight, wherein the method comprises administering to a human subject in need thereof a therapeutically effective amount of a composition comprising a GLP-1 peptide, wherein the GLP-1 peptide is GLP-1 (7-36), wherein the therapeutically effective amount of the GLP-1 peptide is from 0.11 mg/day to 0.66 mg/day and wherein the composition is administered by two or three injections per day.

7. The cosmetic method of claim 6, wherein the subject is a male subject.

8. The cosmetic method of any one of claims 6-7, wherein the therapeutically effective amount of GLP-1 (7-36) corresponds to 0.33 mg/day to 0.66 mg/day.

9. The cosmetic method of claim 6, wherein the subject is a female subject.

10. The cosmetic method of any one of claims 6-9, wherein the composition is administered by parenteral injection, intravenous injection or subcutaneous injection.

## Patentansprüche

1. Zusammensetzung, umfassend ein GLP-1-Peptid zur Verwendung in einem Verfahren zur Behandlung von Adipositas, wobei das Verfahren das Verabreichen einer therapeutisch wirksamen Menge einer Zusammensetzung, die ein GLP-1-Peptid umfasst, an ein menschliches Individuum, das an Adipositas leidet, umfasst, wobei das GLP-1-Peptid GLP-1 (7-36) ist, wobei die therapeutisch wirksame Menge des GLP-1-Peptids 0,11 mg/Tag bis 0,66 mg/Tag beträgt und wobei die Zusammensetzung durch zwei oder drei Injektionen pro Tag verabreicht wird.

2. Zusammensetzung zur Verwendung nach Anspruch 1, wobei das Individuum ein männliches Individuum ist.

3. Zusammensetzung zur Verwendung nach einem der vorangehenden Ansprüche, wobei die therapeutisch wirksame Menge des GLP-1-Peptids 0,33 mg/Tag bis 0,66 mg/Tag beträgt.

4. Zusammensetzung zur Verwendung nach Anspruch 1, wobei das Individuum ein weibliches Individuum ist.

5. Zusammensetzung zur Verwendung nach einem der vorangehenden Ansprüche, wobei die Zusammensetzung durch parenterale Injektion, intravenöse Injektion oder subkutane Injektion verabreicht wird.

6. Kosmetisches Verfahren zum Management des Körpergewichts, wobei das Verfahren das Verabreichen einer therapeutisch wirksamen Menge einer Zusammensetzung, die ein GLP-1-Peptid umfasst, an ein menschliches Individuum, das dessen bedarf, umfasst, wobei das GLP-1-Peptid GLP-1 (7-36) ist, wobei die therapeutisch wirksame Menge des GLP-1-Peptids 0,11 mg/Tag bis 0,66 mg/Tag beträgt und wobei die Zusammensetzung durch zwei oder drei Injektionen pro Tag verabreicht wird.

7. Kosmetisches Verfahren nach Anspruch 6, wobei das Individuum ein männliches Individuum ist.

8. Kosmetisches Verfahren nach einem der Ansprüche 6-7, wobei die therapeutisch wirksame Menge an GLP-1 (7-36) 0,33 mg/Tag bis 0,66 mg/Tag entspricht.

9. Kosmetisches Verfahren nach Anspruch 6, wobei das Individuum ein weibliches Individuum ist.

10. Kosmetisches Verfahren nach einem der Ansprüche 6-9, wobei die Zusammensetzung durch parenterale Injektion, intravenöse Injektion oder subkutane Injektion verabreicht wird.

## Revendications

1. Composition comprenant un peptide GLP-1 destiné à être utilisé dans un procédé de traitement de l'obésité, où le procédé comprend l'administration à un sujet humain souffrant d'obésité d'une quantité thérapeutiquement efficace d'une composition comprenant un peptide GLP-1, où le peptide GLP-1 est le GLP-1 (7-36), où la quantité thérapeutiquement efficace du peptide GLP-1 est de 0,11 mg/jour à 0,66 mg/jour, et où la composition est administrée au moyen de deux ou trois injections par jour.

2. Composition selon la revendication 1, où le sujet est un sujet de sexe masculin.

3. Composition selon l'une quelconque des revendications précédentes, où la quantité thérapeutiquement efficace du peptide GLP-1 est de 0,33 mg/jour à 0,66 mg/jour.

4. Composition selon la revendication 1, où le sujet est un sujet de sexe féminin.

5. Composition selon l'une quelconque des revendications précédentes, où la composition est administrée par injection parentérale, injection intraveineuse ou injection sous-cutanée.

6. Procédé cosmétique de gestion du poids corporel, où le procédé comprend l'administration à un sujet humain qui en a besoin d'une quantité thérapeutiquement efficace d'une composition comprenant un peptide GLP-1, où le peptide GLP-1 est le GLP-1 (7-36), où la quantité thérapeutiquement efficace du peptide GLP-1 est de 0,11 mg/jour à 0,66 mg/jour, et où la composition est administrée au moyen de deux ou trois injections par jour.

7. Procédé cosmétique selon la revendication 6, où le sujet est un sujet de sexe masculin.

8. Procédé cosmétique selon l'une quelconque des revendications 6 à 7, où la quantité thérapeutiquement efficace du peptide GLP-1 (7-36) est de 0,33 mg/jour à 0,66 mg/jour.

9. Procédé cosmétique selon la revendication 6, où le sujet est un sujet de sexe féminin.

10. Procédé cosmétique selon l'une quelconque des revendications 6 à 9, où la composition est administrée par injection parentérale, injection intraveineuse ou injection sous-cutanée.
